(19) Europäisches Patentamt
European Patent Office
Office européen des brevets

(11) EP 3 555 762 B1

(12) FASCICULE DE BREVET EUROPEEN

(45) Date de publication et mention
de la délivrance du brevet:
**23.08.2023 Bulletin 2023/34**

(51) Classification Internationale des Brevets (IPC):
**G06F 17/11** (2006.01)    **F02M 21/02** (2006.01)
**F02M 21/06** (2006.01)    **F02B 43/10** (2006.01)

(21) Numéro de dépôt: **17825593.1**

(22) Date de dépôt: **15.12.2017**

(52) Classification Coopérative des Brevets (CPC):
**G06F 17/11; F02M 21/0215; G01N 33/225**

(86) Numéro de dépôt international:
**PCT/FR2017/053615**

(87) Numéro de publication internationale:
**WO 2018/109418 (21.06.2018 Gazette 2018/25)**

(54) **PROCÉDÉ POUR CALCULER EN TEMPS RÉEL L'INDICE DE MÉTHANE MN DE LA PHASE LIQUIDE D'UN GAZ NATUREL LIQUÉFIE**

VERFAHREN ZUR ECHTZEIT-BERECHNUNG DER METHANZAHL MZ IN DER FLÜSSIGPHASE VON VERFLÜSSIGTEM ERDGAS

METHOD FOR CALCULATING IN REAL TIME THE METHANE NUMBER MN IN THE LIQUID PHASE OF A LIQUEFIED NATURAL GAS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.12.2016 FR 1662507**

(43) Date de publication de la demande:
**23.10.2019 Bulletin 2019/43**

(73) Titulaire: **ENGIE**
**92400 Courbevoie (FR)**

(72) Inventeur: **BEN BELGACEM - STREK, Michel**
**75018 Paris (FR)**

(74) Mandataire: **Novagraaf Technologies**
**Bâtiment O2**
**2, rue Sarah Bernhardt**
**CS90017**
**92665 Asnières-sur-Seine Cedex (FR)**

(56) Documents cités:
**EP-A1- 2 984 385       WO-A1-2016/097651
KR-A- 20160 086 438    US-A- 5 333 591**

• **Paw Andersen: "Algorithm for methane number determination for natural gasses", Project Report 719.45, 3 juin 1999 (1999-06-03), pages 1-29, XP055407334, ISBN: 978-87-7795-125-1 Extrait de l'Internet: URL:http://www.dgc.dk/sites/default/files/ filer/publikationer/R9907_algorithm_methan e.pdf [extrait le 2017-09-18]**
• **LOTTERS J C ET AL: "Real-time composition determination of gas mixtures", IEEE SENSORS 2014 PROCEEDINGS, IEEE, 2 novembre 2014 (2014-11-02), pages 1640-1643, XP032705359, DOI: 10.1109/ICSENS.2014.6985334**
• **Karine Arrhenius ET AL: "Method development for gas quality determination in the LNG storage of a LNG/LCNG refuelling station", SGC Rapport 2013:288, septembre 2013 (2013-09), pages 1-42, XP055407305, Malmö, Sweden Extrait de l'Internet: URL:https://www.sp.se/sv/training/Document s/SGC%202013-288.pdf [extrait le 2017-09-18]**
• **GIESEKING BJÖRN ET AL: "Novel algorithm for calculating the methane number of liquefied natural gas with defined uncertainty", FUEL, IPC SCIENCE AND TECHNOLOGY PRESS, GUILDFORD, GB, vol. 185, 20 août 2016 (2016-08-20), pages 932-940, XP029706836, ISSN: 0016-2361, DOI: 10.1016/J.FUEL.2016.07.105**

- Classnk / Nippon ET AL: "LNG Fuelled Ships - Activities of ClassNK -", LNG-Fuelled Vessel Technologies Seminar Activities of ClassNK, 25 juillet 2014 (2014-07-25), XP055206873, Extrait de l'Internet: URL:http://classnk-rd.com/LNG-Fuelled_Vessel_Technologies_Seminar/pdf/6_Activities_of_ClassNK-LNG_Fuelled_Ship-.pdf [extrait le 2015-08-07]

**Description**

La présente invention se rapporte de manière générale à un procédé de calcul en temps réel de l'indice de méthane d'un gaz naturel liquéfié contenu dans un réservoir, et notamment dans un réservoir embarqué.

Le GNL carburant est une alternative simple et efficace aux combustibles classiques, tant du point de vue de l'émission de $CO_2$, de particules polluantes que de la masse volumique énergétique. De plus en plus d'acteurs se tournent vers son utilisation, comme les transporteurs routiers, maritimes ou ferroviaires.

Cependant, contrairement aux carburants classiques raffinés, le GNL ne possède pas une composition standard. Selon l'emplacement du gisement, le GNL peut contenir plus au moins de composés lourds (C2, C3, C4, ...) .

La variance de la composition implique une difficulté : le phénomène de cliquetis (ou « *knocking* » en anglais) peut se déclarer dans les moteurs lorsque la part des composés lourds est importante ; il est dû à une mauvaise combustion déclenchée par des hydrocarbures lourds à un mauvais moment au mauvais endroit, donnant lieu à un deuxième front de combustion: d'où l'arrivée de problèmes de performance, de rendement et de tenue mécanique, voire d'arrêts moteur, etc. L'indice de méthane est le paramètre permettant de prévoir l'apparition de ce phénomène et d'enclencher les actions correctrices nécessaires.

Pour résoudre ce problème d'appariation du phénomène de cliquetis, l'indice de méthane doit être le plus stable possible. Or, l'indice de méthane est aujourd'hui une grandeur mal connue et dont les méthodes de détermination sont très diverses quant au résultat. La méthode actuellement d'usage consistant à calculer un indice de méthane à partir d'une composition de GNL reçue au terminal reste donc une vue de l'esprit, sachant de plus que cet indice varie avec le vieillissement, selon le procédé de soutage du réservoir utilisé et selon la règle de calcul choisie. La capacité de déterminer l'indice de méthane est donc un enjeu primordial pour démocratiser et favoriser l'essor du marché du GNL carburant au détail.

En l'état actuel de l'art, seule la détection d'un premier cliquetis permet de déceler une dérive de l'indice de méthane permettant d'opérer ensuite aux corrections nécessaires : la technique actuellement employée est la détection de cliquetis par capteur sonore, ce qui déclenche une modification du régime moteur pour adapter l'entrée d'air et prévenir les prochains cliquetis.

Pour déterminer l'indice de méthane d'un GNL, il est connu de l'homme de l'art des solutions basées sur la détermination de la composition de la phase gazeuse qui permettent d'approcher l'indice de méthane. Cette détermination de la phase gazeuse peut notamment être réalisée comme suit :

- par mesure optique par spectrométrie : cela permet une précision suffisante sur les hydrocarbures lourds, mais pas sur l'azote qui est utilisé pour couper le gaz en vue de maîtriser l'indice de méthane (la spectrométrie mesure la réponse spectrale des seules liaisons CH). De plus, une telle méthode de détermination de la composition n'est utilisée aujourd'hui que pour des moteurs à essence (il existe donc encore une incertitude sur sa capacité à répondre au besoin du GNL). Enfin, une telle méthode est très coûteuse car le coût du capteur optique est de l'ordre de 100 USD;
- par calcul par corrélation empirique avec des capteurs de température et de pression : le coût d'une telle solution représente, à l'heure actuelle, plus du tiers du cout d'une cuve GNL, ce qui est assez dissuasif. En outre, les capteurs de température et de pression actuellement connus sont typiquement destinés à des moteurs à essence et devraient, le cas échéant, être encore adaptés au GNL ;
- par mesure par chromatographie : l'encombrement et le coût de l'équipement rendent cette solution inenvisageable avec un réservoir embarqué.

Afin de pallier les inconvénients précités, le déposant a mis en place un procédé pour calculer en temps réel l'indice de méthane MN d'un gaz naturel liquéfié (généralement désigné par l'acronyme GNL) contenu dans un réservoir tel qu'un réservoir embarqué. Ce procédé de calcul de l'indice de méthane est basé sur la détermination de la composition d'un GNL en phase liquide multi-composants, qui n'utilise de manière avantageuse que trois types de capteurs différents. Cette détermination est ainsi économique et n'implique pas de mesures en phase gazeuse.

Les solutions connues de l'art antérieur (citées précédemment) cherchent à approcher la valeur de l'indice de méthane à partir de la détermination de la composition de la phase gazeuse ; elles trouvent donc des valeurs qui ne correspondent pas à la qualité réelle du gaz injecté dans le moteur. En effet, la composition de la phase gazeuse est différente de celle de la phase liquide à cause des différences de volatilité des composants.

Or, les technologies actuelles d'injection privilégient la vaporisation du GNL liquide. C'est donc cette composition de la phase liquide qui doit être approchée. Une fois la composition connue, il devient simple d'approcher l'indice de méthane par un calcul dépendant de la norme choisie. Plusieurs algorithmes sont connus pour calculer l'indice de méthane MN d'un gaz, dont par exemple celui décrit dans le document Paw Andersen : "Algorithm for méthane number dermination for natural gasses", Project Report 719.45, 3 juin 1999 (1999-06-03).

Plus particulièrement, la présente invention a donc pour objet un procédé pour calculer en temps réel l'indice de méthane MN d'un gaz naturel liquéfié contenu dans un réservoir contenant du gaz naturel 4se répartissant en :

- une couche de gaz naturel (11) à l'état liquide (1) définie à un instant t donné par sa température T(t) et sa masse volumique ρ(t), ladite couche de gaz naturel (11) étant en équilibre avec
- une couche de gaz naturel (12) à l'état gazeux (g), ledit procédé étant caractérisé en ce qu'il consiste en un algorithme comportant, à un instant t donné, les étapes suivantes :

A. détermination, par mesure, de la température $T_0(t)$ et de la masse volumique $\rho_0(t)$ de la couche de gaz naturel à l'état liquide, ainsi que de la pression $P_0(t)$ de la couche de gaz naturel à l'état gazeux (g) ;

B. calcul approché de la composition du gaz naturel liquéfié contenu dans le réservoir, par un algorithme de calcul de minimum sous contrainte, ledit algorithme comprenant les sous-étapes suivantes :

b1) détermination, par calcul de minimum sous contraintes, d'une première composition de masse volumique $\rho_0(t)$ et à pression $P_0(t)$, ou d'une première enveloppe de compositions présentant la même masse volumique $\rho_0(t)$ à pression $P_0(t)$ ;

b2) détermination, par calcul de minimum sous contraintes, d'une deuxième enveloppe de compositions présentant la même masse volumique $\rho_0(t)$ à température $T_0(t)$ et à pression $P_0(t)$, et dont la température d'équilibre $T_{eq}(t)$ à pression $P_0(t)$ est égale à $T_0(t)$ ;

b3) détermination d'un point non singulier de la deuxième enveloppe donnant une composition approchante de la composition réelle du GNL ; et

b4) calcul de l'indice de méthane MN à partir de ladite composition approchante.

Le procédé de calcul selon l'invention est implémenté par ordinateur.

Par réservoir, on entend, au sens de la présente invention, une cuve pressurisée ou à pression ambiante, du type méthanier ou terminal gazier.

Par température d'équilibre $T_{eq}$ (ou température de bulle) on entend, au sens de la présente invention, la température à partir de laquelle les premières bulles apparaissent quand on chauffe, à la pression du ciel gazeux.

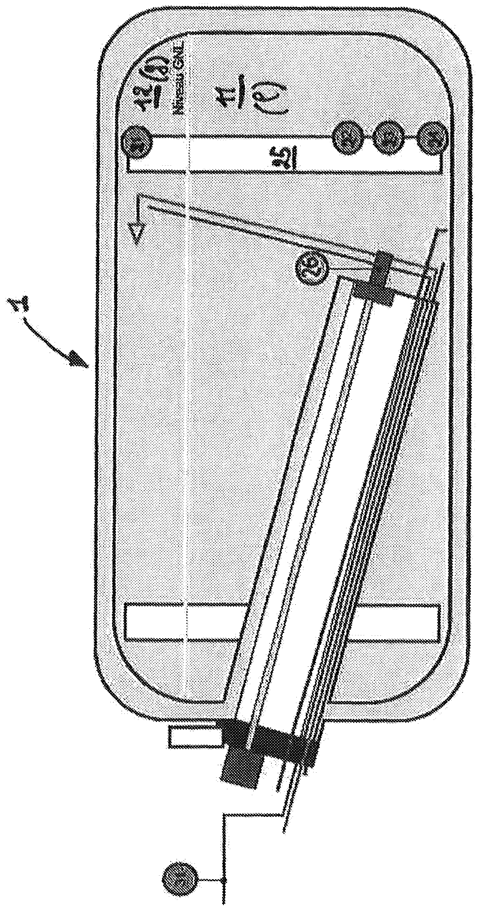

Dans le cadre du procédé selon l'invention et en particulier de l'étape A, la mesure de la température T0(t) est réalisée à l'aide d'au moins un capteur de température. Toutefois, afin de s'assurer de l'homogénéité de la mesure dans la cuve de GNL, il est possible d'utiliser avantageusement plusieurs capteurs de températures, par exemple un capteur dans le ciel gazeux et trois capteurs dans couche de gaz naturel à l'état liquide, comme illustré sur la figure 1 (cf. exemple 1).

Dans le cadre de l'étape A du procédé selon l'invention, la mesure de la masse volumique $\rho_0(t)$ de la couche de gaz naturel à l'état liquide est réalisée à l'aide d'au moins un capteur de masse volumique. Un capteur unique peut suffire pour la mesure de la masse volumique, le GNL étant relativement homogène en termes de composition et de température dans un réservoir de stockage.

Dans le cadre de l'étape A du procédé selon l'invention, la mesure de la pression $P_0(t)$ de la couche de gaz naturel à l'état gazeux (g) est réalisée à l'aide d'au moins un capteur de pression. Un capteur unique peut suffire pour la mesure de la pression, la pression étant homogène dans la phase gazeuse.

Le procédé selon l'invention présente l'avantage de pouvoir approcher la composition d'un GNL, avec une très faible marge d'erreur (inférieure à 5%), à partir de mesures réalisées par trois types de capteurs différents (notamment la température et la masse volumique de la phase liquide du gaz naturel, et la pression de sa phase gazeuse).

En effet, si l'on part de l'hypothèse que le GNL, dont on cherche à déterminer la composition, et par suite l'indice MN, contient jusqu'à des composés en C5 et de l'azote, on peut dénombrer classiquement 9 éléments le composant, à savoir : $CH_4$, $C_2H_6$, $C_3H_8$, $iC_4H_{10}$, $nC_4H_{10}$, $iC_5H_{12}$, $nC_5H_{12}$, $C_6H_{14}$ et $N_2$. En supposant qu'une grandeur physique fournisse une équation reliant les fractions molaires de chaque composant, il serait nécessaire d'obtenir 8 informations différentes (la fraction du dernier composant se déduisant des 8 autres).

A cette fin, le procédé selon l'invention part des trois paramètres clés mesurables que sont la température, la masse volumique de la phase liquide du GNL et la pression du gaz, auxquels il ajoute des hypothèses de condition « statistique » idéale (telle que rencontrée dans l'industrie du GNL et définie dans les publications du GIIGNL (acronyme désignant l' « *International Group of Liquefied Natural Gas Importers* »)[1]), et utilise un algorithme d'optimisation non linéaire mis en oeuvre par un calculateur (par exemple un microprocesseur ou un PC) afin de résoudre l'équation (reliant les fractions molaires de chaque composant) en vue d'obtenir l'indice de méthane.

L'étape principale du procédé selon l'invention est l'étape B de calcul approché de la composition du gaz naturel liquéfié contenu dans le réservoir par un algorithme de calcul de minimum sous contrainte mis en oeuvre par un calculateur. Dès qu'un utilisateur souhaite connaître les caractéristiques d'un GNL (composition, indice MN), le calculateur exécute l'algorithme de afin de déterminer la composition du GNL à partir de la température, de la pression et de la masse volumique.

Ce calcul repose sur une hypothèse essentielle : le GNL est en équilibre avec sa phase gazeuse, c'est-à-dire que le liquide se situe thermodynamiquement sur sa courbe de bulle. Cette supposition est la clé de voute du calcul.

En effet, supposer le GNL en équilibre (ce qui est le cas la plupart du temps dans un stockage de GNL) permet de mettre en relation la température, la pression et la composition du GNL. Cette équation et la valeur de la masse volumique (dépendant de la composition) forment alors un système à résoudre afin de déterminer la composition.

L'algorithme du procédé selon l'invention permet de donner en trois étapes (étapes b1 à b3) un ensemble de compositions possibles du GNL :

- on détermine d'abord une composition de GNL ou une enveloppe de compositions de GNL présentant la masse volumique $\rho_0(t)$ mesurée à la pression mesurée $P_0(t)$ (étape b1) ; puis
- à partir de la composition ou enveloppe de compositions calculées à l'étape b1, on détermine une deuxième enveloppe de compositions dont la température d'équilibre $T_{eq}(t)$ à pression $P_0(t)$ est égale à la température mesurée $T_0(t)$ (étape b2) ;
- on détermine alors un point non singulier donnant une composition approchante de la composition réelle du GNL (étape b3) .

Afin de réduire l'espace des solutions (i.e. les compositions possibles) obtenues à l'issue de chacune des étapes b1 et b2, celles-ci sont des étapes de calcul de minimum sous contrainte de type gradient (gradient conjugué, Newton projeté, point intérieur, ...) .

L'indice de méthane MN est ensuite calculé (étape b4) à partir de la composition approchante obtenue à l'étape b3. Ce calcul est simple et utilise un des calculs standard de l'industrie du GNL (méthode AVL de l'entreprise éponyme, méthode CARB proposée par SAE International, méthode GRI du Gas Research Institute,...) .

Quelle que soit la méthode de calcul utilisée, l'indice de méthane est un nombre adimensionnel compris entre 0 et 100, la valeur « 0 » correspondant en théorie à une composition comprenant 100% d'hydrogène et la valeur « 100 » correspondant à une composition comprenant 100% de méthane.

Pour une erreur de calcul sur la composition approchante de l'ordre de 5 à 10%, il est possible d'atteindre une précision sur la valeur de l'indice de méthane inférieure à 0,2% en fonction des conditions physiques de température et de pression.

Selon un premier mode de réalisation avantageux de l'étape b1) du procédé selon l'invention la détermination de la première enveloppe de compositions peut être réalisée comme suit en cherchant à résoudre les équations suivantes :

$$\min \rho(x, P\circ) - \rho_0$$
$$s.t \begin{cases} \rho(x, P\circ) - \rho_0 \geq 0 \\ \sum x_i = 1 \\ x_i^l \leq x_i \leq x_i^u \end{cases}$$

avec :

ρ désignant la masse volumique calculée à partir de x et $P_0$
$\rho_0$ désignant la masse volumique mesurée,
$P_0$ désignant la pression mesurée,
x désignant le vecteur composition, composé de $x_i$
$x_i$ désignant la fraction molaire du composant i, les exposants l et u faisant respectivement référence à la limite inférieure et supérieure de cette fraction molaire.

Selon un deuxième mode de réalisation avantageux de l'étape b1) du procédé selon l'invention, la détermination de la première enveloppe de compositions peut également être réalisée comme suit en cherchant à résoudre les équations suivantes :

$$\min((\rho(x, P\circ) - \rho_0)^2)$$
$$s.t \begin{cases} \sum x_i = 1 \\ x_i^l \leq x_i \leq x_i^u \end{cases}$$

avec :

$\rho$ désignant la masse volumique calculée à partir de x et $P_0$
$\rho_0$ désignant la masse volumique mesurée,
$P_0$ désignant la pression mesurée,
x désignant le vecteur composition, composé de $x_i$
$x_i$ désignant la fraction molaire du composant i, les exposants l et u faisant respectivement référence à la limite inférieure et supérieure de cette fraction molaire.

Il est évident pour l'homme du métier de trouver d'autres modes de réalisation de l'étape b1) à partir des deux modes avantageux décrits ci-dessus, en utilisant d'autres types connus de calcul de minimum sous contrainte.

Selon un premier mode de réalisation avantageux de l'étape b2) du procédé selon l'invention la détermination de la deuxième enveloppe de compositions peut être réalisée comme suit en cherchant à résoudre les équations suivantes :

$$\min T_{eq}(x, P\circ) - T_0$$

$$s.t \begin{cases} T_{eq}(x, P\circ) - T_0 \geq 0 \\ \rho(x, P\circ) - \rho_0 = 0 \\ \sum x_i = 1 \\ x_i^l \leq x_i \leq x_i^u \end{cases}$$

avec :

$T_0$ désignant la température mesurée,
$T_{eq}$ désignant la température d'équilibre du GNL à pression $P_0$,
$\rho$, $\rho_0$, $P_0$, x, $x_i$, et u étant tels que définis précédemment.

Selon un deuxième mode de réalisation avantageux de l'étape b2) du procédé selon l'invention, la détermination de la deuxième enveloppe de compositions peut être réalisée comme suit en cherchant à résoudre les équations suivantes :

$$\min((T_{eq}(x, P\circ) - T_0)^2)$$

$$s.t \begin{cases} \rho(x, P\circ) - \rho_0 = 0 \\ \sum x_i = 1 \\ x_i^l \leq x_i \leq x_i^u \end{cases}$$

avec :

$T_0$ désignant la température mesurée,
$T_{eq}$ désignant la température d'équilibre du GNL à pression $P_0$,
$\rho$, $\rho_0$, $P_0$, x, $x_i$, et u étant tels que définis précédemment.

Il est évident pour l'homme du métier de trouver d'autres modes de réalisation de l'étape b2) à partir des deux modes avantageux décrits ci-dessus, en utilisant d'autres types connus de calcul de minimum sous contrainte.

Selon un premier mode de réalisation avantageux de l'étape b3) du procédé selon l'invention, le point non singulier de la deuxième enveloppe peut être le barycentre de la deuxième enveloppe ou le point de la deuxième enveloppe qui est le plus proche du barycentre de la deuxième enveloppe.

Selon un deuxième mode de réalisation avantageux de l'étape b3) du procédé selon l'invention, le point non singulier de la deuxième enveloppe peut être calculé comme la moyenne renormalisée des compositions normalisées de la deuxième enveloppe.

D'autres modes de réalisation de l'étape b3) sont possibles à partir des deux modes avantageux décrits ci-dessus, en utilisant d'autres types connus de points singuliers.

D'autres avantages et particularités de la présente invention résulteront de la description qui va suivre, donnée à titre d'exemple non limitatif et faite en référence à la figure unique annexée, représentant schématiquement un exemple de réservoir (ou cuve) contenant du gaz naturel avec différents capteurs de température, masse volumique et pression.

Plus particulièrement, cette figure montre une cuve 1 horizontale contenant du gaz naturel se répartissant en :

- une couche de gaz naturel 11 à l'état liquide l, définie à un instant t donné, par sa température $T(t)$ et sa masse volumique $\rho(t)$, cette couche de gaz naturel 11 à l'état liquide étant en équilibre avec
- une couche de gaz naturel 12 à l'état gazeux (g), définie à un instant t donné par sa pression $P(t)$.

La cuve 1 comporte :

- une pluralité de capteurs de température 21, 22, 23, 24 pour déterminer la température $T_0(t)$ du GNL, ces capteurs sont disposés sur un support 25, l'un d'entre eux 21 se situant dans la phase gazeuse (ou ciel gazeux) du GNL, et trois d'entre eux se situent dans la phase liquide (la température du gaz n'est pas prise en compte dans le calcul),
- un capteur de masse volumique 26 pour déterminer la masse volumique $\rho_0(t)$ du GNL à l'état liquide (un seul capteur est suffisant car le GNL à l'état liquide présente une densité homogène), et
- un capteur de pression 31 pour déterminer la pression $P_0(t)$ du ciel gazeux.

Une fois mesurées à chaque instant les valeurs de température $T_0(t)$, de masse volumique $\rho_0(t)$ et de pression $P_0(t)$, celles-ci sont injectées dans un calculateur mettant en oeuvre le procédé selon l'invention pour définir une composition approchante du GNL. Les résultats de ces simulations sont présentés dans l'exemple 1 ci-après.

Le procédé de calcul selon l'invention illustrée plus en détail dans l'exemple ci-après. L'invention est définie dans la revendication 1.

**EXEMPLE**

L'exemple ci-dessous est réalisé avec nombre limité de points (enveloppe de 6 compositions) à des fins pédagogiques et afin d'en faciliter la lecture et la compréhension du raisonnement par l'homme du métier. Celui-ci pourra aisément comprendre par déduction simple qu'en augmentant le nombre de points de mesure on arrivera à un résultat de l'indice dont la précision augmentera avec le nombre de points de mesure.

Les paramètres d'entrée du procédé selon l'invention sont les suivants :

- $T_0(t)$ = -160,76°C ;
- $\rho_0(t)$ = 448,11 g/cm3 ;
- $P_0(t)$ = 1190 mbar.

A partir de ces valeurs, on détermine alors une composition de GNL présentant la masse volumique $\rho_0(t)$ mesurée à la pression mesurée $P_0(t)$ (étape b1). Cette première composition est donnée dans le tableau 1 ci-après.

Tableau 1 : première composition

| Composants du GNL | % mol (première composition) |
|---|---|
| $CH_4$ | 91, 09 |
| $C_2H_6$ | 7,14 |
| $C_3H_8$ | 1,54 |
| $iC_4$ | 0,117 |
| $nC_4$ | 0,01 |
| $iC_5$ | 0 |
| $nC_5$ | 0 |
| $C_6$ | 0 |
| $N_2$ | 0 |

Pour cette première composition, on a calculé la température d'équilibre par une méthode de calcul classique d'enveloppe de phase (par exemple la méthode de Rachford-Rice)[2], qui est de - 158,36°C. La masse volumique $\rho(t)$ est recalculée pour vérification : $\rho(t)$ = 448,11 g/cm3 pour $T_0(t)$ = -160,76°C.

Puis, à partir de cette première composition calculée à l'étape b1, on détermine une enveloppe de compositions dont la température d'équilibre $T_{eq}(t)$ à pression $P_0(t)$ est égale à la température mesurée $T_0(t)$, soit -160,76°C (étape b2). Cette enveloppe comporte six compositions C1 à C6, qui sont détaillées dans le tableau 2 ci-après :

Tableau 2 : enveloppe de compositions pour laquelle $T_{eq}(t)=T_0(t)$

| Composition du GNL | % mol (C1) | % mol (C2) | % mol (C3) | % mol (C4) | % mol (C5) | % mol (C6) |
|---|---|---|---|---|---|---|
| $CH_4$ | 91,89 | 90,07 | 92,56 | 91,8 | 92,57 | 92,14 |
| $C_2H_6$ | 5,97 | 8,52 | 5 | 6,16 | 5 | 5,7 |
| $C_3H_8$ | 1,45 | 0,267 | 1,22 | 1,35 | 1,22 | 1,15 |
| $iC_4$ | 0 | 0 | 0,27 | 0,002 | 0,27 | 1,15 |
| $nC_4$ | 0 | 0 | 0,27 | 0,002 | 0,27 | 0 |
| $iC_5$ | 0 | 0 | 0 | 0 | 0 | 0 |
| $nC_5$ | 0 | 0 | 0 | 0 | 0 | 0 |
| $C_6$ | 0 | 0 | 0 | 0 | 0 | 0 |
| $N_2$ | 0,679 | 0,721 | 0,654 | 0,682 | 0,654 | 0,67 |

Pour cette enveloppe de compositions C1 à C6, on a calculé la température d'équilibre (également par une méthode de calcul classique d'enveloppe de phase telle que la méthode de Rachford-Rice)[2] ) : $T_{eq}(t)$= -160,76°C, soit la température $T_0$ mesurée, ce qui montre que le calcul est réalisé correctement.

A partir de cette enveloppe de compositions C1 à C6, on détermine un point non singulier de la deuxième enveloppe donnant une composition approchante de la composition réelle du GNL. L'indice de méthane MN est ensuite calculé (étape b4) à partir de la composition approchante obtenue à l'étape b3.

Dans une premier cas, on prend comme point non singulier le barycentre donnant une composition approchante de la composition réelle du GNL (étape b3), donnée dans le tableau 3 ci-après :

Tableau 3 : composition du barycentre

| Composants du GNL | % mol |
|---|---|
| $CH_4$ | 91,8383333 |
| $C_2H_6$ | 6,05833333 |
| $C_3H_8$ | 1,1095 |
| $iC_4$ | 0,282 |
| $nC_4$ | 0,09033333 |
| $iC_5$ | 0 |
| $nC_5$ | 0 |
| $C_6$ | 0 |
| $N_2$ | 0,67666667 |

L'indice de méthane MN est ensuite calculé (étape b4) à partir de la composition approchante obtenue à l'étape b3. Ce calcul est simple et utilise des méthodes de calcul standard de l'industrie du GNL (CARB, GRI H/C, GRI coefficient linéaire, et MON/KIWA, MWM, AVL), qui sont des méthodes empiriques bien connues de l'homme de l'art basées sur différentes expériences et types de moteur. Les méthodes MWM et AVL sont les méthodes publiques les plus communément utilisées dans l'industrie.

Les résultats de ces calculs sont indiqués dans le tableau 4 ci-dessous pour chacune des différentes méthodes de calcul de l'indice de méthane, à partir de la composition du tableau 3.

Tableau 4 : indice de méthane MN

| | |
|---|---|
| MN (Méthode CARB) | 86,9427269 |
| MN (Méthode GRI H/C) | 79, 9123525 |
| MN (Méthode GRI coeff. linéaire) | 81,1445809 |
| Méthode MON/KIWA | 78,9080193 |
| Méthode MWM | 78 |
| Méthode AVL | 78,7 |

On remarque que les différentes méthodes donnent des résultats différents. Ceci est normal car n'y a pas d'accord aujourd'hui dans l'industrie sur la méthode à utiliser pour calculer l'indice de méthane. Différentes méthodes existent, et chaque société a une méthode de prédilection. D'autres méthodes de calcul, moins répandues, existent et peuvent tout aussi bien être utilisées à partir de la composition précédemment déterminée.

Dans un deuxième cas, à partir de l'enveloppe de compositions C1 à C6 obtenue précédemment, on prend comme point non singulier la moyenne renormalisée des compositions normalisées de la deuxième enveloppe, dont la composition est donnée dans le tableau 5 ci-après :

Tableau 5: deuxième composition

| Composants du GNL | % mol |
|---|---|
| $CH_4$ | 91,7873393 |
| $C_2H_6$ | 6,05721857 |
| $C_3H_8$ | 1,10826957 |
| $iC_4$ | 0,28047889 |
| $nC_4$ | 0,09035225 |
| $iC_5$ | 0 |
| $nC_5$ | 0 |
| $C_6$ | 0 |
| $N_2$ | 0,67634147 |

L'indice de méthane MN est ensuite calculé (étape b4) à partir de cette composition approchante obtenue à l'étape b3 (donnée dans le tableau 5) en utilisant les méthodes de calcul standard de l'industrie du GNL décrites précédemment (MWM, AVL). Les résultats des calculs d'indice de méthane sont indiqués dans le tableau 6 ci-dessous pour chacune des différentes méthodes utilisées, à partir de la composition du tableau 5.

Tableau 6 : indice de méthane MN

| | |
|---|---|
| MN (Méthode CARB) | 86,9479438 |
| MN (Méthode GRI H/C) | 79,9169944 |
| MN (Méthode GRI coeff. linéaire) | 81,0463815 |
| Méthode MON/KIWA | 78,9166216 |
| Méthode MWM | 77 |
| Méthode AVL | 78,7 |

On constate que les valeurs calculées dans le tableau 6 sont proches de celles du tableau 4.

Tous les écarts entre le tableau 4 et le tableau 6 sont inférieurs à 0,2%, à l'exception de celui pour la méthode MWM, de 1,3%. La méthode MWM est connue dans l'industrie pour ne pas être une méthode stable et donner des écarts de 1 en absolu pour des compositions très proches, donc ce résultat pouvait être attendu.

## LISTE DES REFERENCES

[1] http://www.giignl.org/publications

[2] Procedure for use of electronic digital computers in calculating flash vaporisation hydrocarbon equilibrium. RACHFORD Jr, H. H., & RICE, J. D. s.l.: Journal of Petroleum Technology, 1952, Vol. 4(10), 19-3.

## Revendications

1. Procédé implémenté par ordinateur pour calculer en temps réel l'indice de méthane MN d'un gaz naturel liquéfié contenu dans un réservoir (1) contenant du gaz naturel se répartissant en :

   - une couche de gaz naturel (11) à l'état liquide (1), définie à un instant t donné, par sa température $T(t)$ et sa masse volumique $\rho(t)$, ladite couche de gaz naturel (11) étant en équilibre avec
   - une couche de gaz naturel (12) à l'état gazeux (g), définie à un instant t donné par sa pression $P(t)$ ;

   ledit procédé étant **caractérisé en ce qu'**il consiste en un algorithme comportant, à un instant t donné, les étapes suivantes :

   A. détermination par mesure de la température $T_0(t)$ et de la masse volumique $\rho_0(t)$ de la couche de gaz naturel (11) à l'état liquide (1), et de la pression $P_0(t)$ de la couche de gaz naturel (12) à l'état gazeux (g) ;
   B. calcul approché de la composition du gaz naturel liquéfié (11) contenu dans le réservoir (1) par un algorithme de calcul de minimum sous contrainte, ledit algorithme comprenant les sous-étapes suivantes :

   b1) détermination, par calcul de minimum sous contraintes, d'une première composition de masse volumique $\rho_0(t)$ et à pression $P_0(t)$, ou d'une première enveloppe de compositions présentant la même masse volumique $\rho_0(t)$ à pression $P_0(t)$ ;
   b2) détermination, par calcul de minimum sous contraintes, d'une deuxième enveloppe de compositions présentant la même masse volumique $\rho_0(t)$ à température $T_0(t)$ et à pression $P_0(t)$, et dont la température d'équilibre $T_{eq}(t)$ à pression $P_0(t)$ est égale à $T_0(t)$ ;
   b3) détermination d'un point non singulier de la deuxième enveloppe donnant une composition approchante de la composition réelle du GNL, le point non singulier de la deuxième enveloppe étant a) le barycentre de la deuxième enveloppe ou le point de la deuxième enveloppe qui est le plus proche du barycentre de la deuxième enveloppe, ou b) étant calculé comme la moyenne renormalisée des compositions normalisées de la deuxième enveloppe, ou c) étant déterminé en utilisant d'autres types de points singuliers à partir dudit barycentre et de ladite moyenne renormalisée ; et
   b4) calcul de l'indice de méthane MN à partir de ladite composition approchante.

2. Procédé selon la revendication 1, dans lequel l'étape b1) de détermination de la première enveloppe de compositions est réalisée comme suit en cherchant à résoudre les équations suivantes :

$$\min \rho(x, P\circ) - \rho_0$$

$$s.t \begin{cases} \rho(x, P\circ) - \rho_0 \geq 0 \\ \sum x_i = 1 \\ x_i^l \leq x_i \leq x_i^u \end{cases}$$

avec :

$\rho$ désignant la masse volumique calculée à partir de x et $P_0$,
$\rho_0$ désignant la masse volumique mesurée, $P_0$ désignant la pression mesurée,
x désignant le vecteur composition, composé de $x_i$,
$x_i$ désignant la fraction molaire du composant i, les exposants l et u faisant respectivement référence à la limite inférieure et supérieure de cette fraction molaire.

3. Procédé selon la revendication 1, dans lequel l'étape b1) de détermination de la première enveloppe de compositions est réalisée comme suit en cherchant à résoudre les équations suivantes :

$$\min((\rho(x,P\circ)-\rho_0)^2)$$

$$s.t \begin{cases} \sum x_i = 1 \\ x_i^l \leq x_i \leq x_i^u \end{cases}$$

avec :

$\rho$ désignant la masse volumique calculée à partir de x et $P_0$,
$\rho_0$ désignant la masse volumique mesurée,
$P_0$ désignant la pression mesurée,
x désignant le vecteur composition, composé de $x_i$,
$x_i$ désignant la fraction molaire du composant i, les exposants l et u faisant respectivement référence à la limite inférieure et supérieure de cette fraction molaire.

4. Procédé selon l'une quelconque des revendications précédentes, dans lequel l'étape b2) de détermination de la deuxième enveloppe de compositions est réalisée comme suit en cherchant à résoudre les équations suivantes :

$$\min T_{eq}(x,P\circ)-T_0$$

$$s.t \begin{cases} T_{eq}(x,P\circ)-T_0 \geq 0 \\ \rho(x,P\circ)-\rho_0 = 0 \\ \sum x_i = 1 \\ x_i^l \leq x_i \leq x_i^u \end{cases}$$

avec :

$T_0$ désignant la température mesurée,
$T_{eq}$ désignant la température d'équilibre du GNL à pression $P_0$,
$\rho$ désignant la masse volumique calculée à partir de x et $P_0$,
$\rho_0$ désignant la masse volumique mesurée,
$P_0$ désignant la pression mesurée,
x désignant le vecteur composition, composé de $x_i$
$x_i$ désignant la fraction molaire du composant i, les exposants l et u faisant respectivement référence à la limite inférieure et supérieure de cette fraction molaire.

5. Procédé selon l'une quelconque des revendications 1 à 3, dans lequel l'étape b2) de détermination de la deuxième enveloppe de compositions est réalisée comme suit en cherchant à résoudre les équations suivantes :

$$\min((T_{eq}(x,P\circ)-T_0)^2)$$

$$s.t \begin{cases} \rho(x,P\circ)-\rho_0 = 0 \\ \sum x_i = 1 \\ x_i^l \leq x_i \leq x_i^u \end{cases}$$

avec :

$T_0$ désignant la température mesurée,

$T_{eq}$ désignant la température d'équilibre du GNL à pression $P_0$,

$\rho$ désignant la masse volumique calculée à partir de x et $P_0$,

$\rho_0$ désignant la masse volumique mesurée,

$P_0$ désignant la pression mesurée,

x désignant le vecteur composition, composé de $x_i$,

$x_i$ désignant la fraction molaire du composant i, les exposants l et u faisant respectivement référence à la limite inférieure et supérieure de cette fraction molaire.

## Patentansprüche

1. Computerimplementiertes Verfahren zum Berechnen einer Methanzahl MN eines verflüssigten natürlichen Gases in Echtzeit, das in einem Behälter (1) enthalten ist, der natürliches Gas enthält, das aufgeteilt ist in:

   - eine Schicht aus natürlichem Gas (11) in flüssigem Zustand (I), die zu einem gegebenen Zeitpunkt t durch ihre Temperatur T(t) und ihre volumenbezogene Masse p(t) definiert ist, wobei die Schicht aus natürlichem Gas (11) im Gleichgewicht ist mit
   - einer Schicht aus natürlichem Gas (12) in gasförmigem Zustand (g), die zu einem gegebenen Zeitpunkt t durch ihren Druck P(t) definiert ist;
   wobei das Verfahren **dadurch gekennzeichnet ist, dass** es aus einem Algorithmus besteht, der zu einem gegebenen Zeitpunkt t die folgenden Schritte aufweist:

   A. Bestimmen der Temperatur $T_0(t)$ und der volumenbezogenen Masse $\rho_0(t)$ der Schicht aus natürlichem Gas (11) in flüssigem Zustand (I) und des Drucks $P_0(t)$ der Schicht aus natürlichem Gas (12) in gasförmigem Zustand (g) durch Messung;
   B. angenähertes Berechnen der Zusammensetzung des verflüssigten natürlichen Gases (11), das in dem Behälter (1) enthalten ist, durch einen Algorithmus zum Berechnen eines Spannungsminimums, der Algorithmus umfassend die folgenden Teilschritte:

      b1) Bestimmen, durch Berechnen des Spannungsminimums, einer ersten Zusammensetzung mit einer volumenbezogenen Masse $\rho_0(t)$ und bei einem Druck $P_0(t)$ oder einer ersten Hülle aus Zusammensetzungen, die die gleiche volumenbezogene Masse $\rho_0(t)$ bei Druck $P_0(t)$ vorweist;
      b2) Bestimmen, durch Berechnen des Spannungsminimums, einer zweiten Hülle aus Zusammensetzungen, die die gleiche volumenbezogene Masse $\rho_0(t)$ bei Temperatur $T_0(t)$ und bei Druck $P_0(t)$ vorweist, und deren Gleichgewichtstemperatur $T_{eq}(t)$ bei Druck $P_0(t)$ gleich $T_0(t)$ ist;
      b3) Bestimmen eines nicht singulären Punkts der zweiten Hülle, der eine angenäherte Zusammensetzung der realen Zusammensetzung des GNL ergibt, wobei der nicht singuläre Punkt der zweiten Hülle ist: a) der Massenmittelpunkt der zweiten Hülle oder der Punkt der zweiten Hülle, der am nächsten zu dem Massenmittelpunkt der zweiten Hülle ist, oder b) berechnet wie der neu normalisierte Mittelpunkt der normalisierten Zusammensetzungen der zweiten Hülle, oder c) bestimmt unter Verwendung von anderen Arten von singulären Punkten von dem Massenmittelpunkt und dem neu normalisierten Mittelpunkt; und
      b4) Berechnen der Methanzahl MN von der angenäherten Zusammensetzung.

2. Verfahren nach Anspruch 1, wobei Schritt b1) des Bestimmens der ersten Hülle der Zusammensetzungen wie folgt durchgeführt wird, indem versucht wird, die folgenden Lösungen aufzulösen:

$$\min \rho(x, P\circ) - \rho_0$$

$$s.t \begin{cases} \rho(x, P\circ) - \rho_0 \geq 0 \\ \sum x_i = 1 \\ x_i^l \leq x_i \leq x_i^u \end{cases}$$

wobei:

p die berechnete volumenbezogene Masse von x und $P_0$ bezeichnet,

$p_0$ die gemessene volumenbezogene Masse bezeichnet,

$P_0$ den gemessenen Druck bezeichnet,

x den Zusammensetzungsvektor bezeichnet, bestehend aus $x_i$,

$x_i$ den Molanteil der Komponente i bezeichnet, wobei die Exponenten I und u jeweils auf den unteren und den oberen Grenzwert dieses Molanteils Bezug nehmen.

3. Verfahren nach Anspruch 1, wobei Schritt b1) des Bestimmens der ersten Hülle der Zusammensetzungen wie folgt durchgeführt wird, indem versucht wird, die folgenden Lösungen aufzulösen:

$$\min((\rho(x, P\circ) - \rho_0)^2)$$

$$s.t\begin{cases} \sum x_i = 1 \\ x_i^l \leq x_i \leq x_i^u \end{cases}$$

wobei:

p die berechnete volumenbezogene Masse von x und $P_0$ bezeichnet,

$\rho_0$ die gemessene volumenbezogene Masse bezeichnet,

$P_0$ den gemessenen Druck bezeichnet,

x den Zusammensetzungsvektor bezeichnet, bestehend aus $x_i$,

$x_i$ den Molanteil der Komponente i bezeichnet, wobei die Exponenten I und u jeweils auf den unteren und den oberen Grenzwert dieses Molanteils Bezug nehmen.

4. Verfahren nach einem der vorstehenden Ansprüche, wobei Schritt b2) des Bestimmens der zweiten Hülle der Zusammensetzungen wie folgt durchgeführt wird, indem versucht wird, die folgenden Lösungen aufzulösen:

$$\min T_{eq}(x, P\circ) - T_0$$

$$s.t\begin{cases} T_{eq}(x, P\circ) - T_0 \geq 0 \\ \rho(x, P\circ) - \rho_0 = 0 \\ \sum x_i = 1 \\ x_i^l \leq x_i \leq x_i^u \end{cases}$$

wobei:

$T_0$ den gemessenen Druck bezeichnet,

$T_{eq}$ die Gleichgewichtstemperatur des GNL bei Druck $P_0$ bezeichnet,

p die berechnete volumenbezogene Masse von x und $P_0$ bezeichnet,

$\rho_0$ die gemessene volumenbezogene Masse bezeichnet,

$P_0$ den gemessenen Druck bezeichnet,

x den Zusammensetzungsvektor bezeichnet, bestehend aus $x_i$,

$x_i$ den Molanteil der Komponente i bezeichnet, wobei die Exponenten I und u jeweils auf den unteren und den oberen Grenzwert dieses Molanteils Bezug nehmen.

5. Verfahren nach einem der Ansprüche 1 bis 3, wobei Schritt b2) des Bestimmens der zweiten Hülle der Zusammensetzungen wie folgt durchgeführt wird, indem versucht wird, die folgenden Lösungen aufzulösen:

$$\min((T_{eq}(x,P\circ)-T_0)^2)$$

$$s.t\begin{cases}\rho(x,P\circ)-\rho_0=0\\\sum x_i=1\\x_i^l\le x_i\le x_i^u\end{cases}$$

wobei:

$T_0$ den gemessenen Druck bezeichnet,
$T_{eq}$ die Gleichgewichtstemperatur des GNL bei Druck $P_0$ bezeichnet,
p die berechnete volumenbezogene Masse von x und $P_0$ bezeichnet,
$p_0$ die gemessene volumenbezogene Masse bezeichnet,
$P_0$ den gemessenen Druck bezeichnet,
x den Zusammensetzungsvektor bezeichnet, bestehend aus $x_i$,
$x_i$ den Molanteil der Komponente i bezeichnet, wobei die Exponenten und u jeweils auf den unteren und den oberen Grenzwert dieses Molanteils Bezug nehmen.

**Claims**

1. A computer-implemented method for calculating, in real time, the methane number MN of a liquefied natural gas contained in a tank (1) containing natural gas distributed into:

   - a natural gas layer (11) in a liquid state (1), defined at a particular time t by its temperature T(t) and its density p(t), said natural gas layer (11) being in equilibrium with
   - a natural gas layer (12) in a gaseous state (g), defined at a particular time t by its pressure P(t);
   said method being **characterized in that** it consists of an algorithm comprising, at a particular time t, the following steps:

   A. determining, by means of measuring, the temperature $T_0(t)$ and density $\rho_0(t)$ of the natural gas layer (11) in the liquid state (1), and the pressure $P_0(t)$ of the natural gas layer (12) in the gaseous state (g);
   B. approximately calculating the composition of the liquefied natural gas (11) contained in the tank (1) by means of a constrained minimum calculation algorithm, said algorithm comprising the following sub-steps:

   b1) determining, by means of constrained minimum calculation, a first composition of density $\rho_0(t)$ and at pressure $P_0(t)$, or a first envelope of compositions which have the same density $\rho_0(t)$ at pressure $P_0(t)$;
   b2) determining, by means of constrained minimum calculation, a second envelope of compositions which have the same density $\rho_0(t)$ at temperature $T_0(t)$ and at pressure $P_0(t)$ and of which the equilibrium temperature $T_{eq}(t)$ at pressure $P_0(t)$ is equal to $T_0(t)$;
   b3) determining a non-singular point of the second envelope to give an approximate composition of the actual composition of the LNG, the non-singular point of the second envelope being a) the barycenter of the second envelope or the point of the second envelope that is closest to the barycenter of the second envelope, or b) being calculated as the renormalized average of the normalized compositions of the second envelope, or c) being determined by using other types of singular points from said barycenter and said renormalized average; and
   b4) calculating the methane number MN from said approximate composition.

2. The method according to claim 1, wherein step b1) of determining the first envelope of compositions is carried out as follows by seeking to solve the following equations:

$$\min \rho(x, P\circ) - \rho_0$$

$$s.t \begin{cases} \rho(x, P\circ) - \rho_0 \geq 0 \\ \sum x_i = 1 \\ x_i^l \leq x_i \leq x_i^u \end{cases}$$

where:

$\rho$ denotes the density calculated from x and $P_0$,
$\rho_0$ denotes the measured density,
$P_0$ denotes the measured pressure,
x denotes the composition vector, composed of $x_i$,
$x_i$ denotes the molar fraction of component i, the exponents I and u referring to the lower limit and upper limit, respectively, of this molar fraction.

3. The method according to claim 1, wherein step b1) of determining the first envelope of compositions is carried out as follows by seeking to solve the following equations:

$$\min((\rho(x, P\circ) - \rho_0)^2)$$

$$s.t \begin{cases} \sum x_i = 1 \\ x_i^l \leq x_i \leq x_i^u \end{cases}$$

where:

$\rho$ denotes the density calculated from x and $P_0$,
$\rho_0$ denotes the measured density,
$P_0$ denotes the measured pressure,
x denotes the composition vector, composed of $x_i$,
$x_i$ denotes the molar fraction of component i, the exponents I and u referring to the lower limit and upper limit, respectively, of this molar fraction.

4. The method according to any of the preceding claims, wherein step b2) of determining the second envelope of compositions is carried out as follows by seeking to solve the following equations:

$$\min T_{eq}(x, P\circ) - T_0$$

$$s.t \begin{cases} T_{eq}(x, P\circ) - T_0 \geq 0 \\ \rho(x, P\circ) - \rho_0 = 0 \\ \sum x_i = 1 \\ x_i^l \leq x_i \leq x_i^u \end{cases}$$

where:

$T_0$ denotes the measured temperature,
$T_{eq}$ denotes the equilibrium temperature of the LNG at pressure $P_0$,
$\rho$ denotes the density calculated from x and $P_0$,
$\rho_0$ denotes the measured density,
$P_0$ denotes the measured pressure,
x denotes the composition vector, composed of $x_i$,

$x_i$ denotes the molar fraction of component i, the exponents l and u referring to the lower limit and upper limit, respectively, of this molar fraction.

5. The method according to any of claims 1 to 3, wherein step b2) of determining the second envelope of compositions is carried out as follows by seeking to solve the following equations:

$$\min((T_{eq}(x, P\circ) - T_0)^2)$$
$$s.t \begin{cases} \rho(x, P\circ) - \rho_0 = 0 \\ \sum x_i = 1 \\ x_i^l \leq x_i \leq x_i^u \end{cases}$$

where:

$T_0$ denotes the measured temperature,
$T_{eq}$ denotes the equilibrium temperature of the LNG at pressure $P_0$,
$\rho$ denotes the density calculated from x and $P_0$,
$\rho_0$ denotes the measured density,
$P_0$ denotes the measured pressure,
x denotes the composition vector, composed of $x_i$,
$x_i$ denotes the molar fraction of component i, the exponents l and u referring to the lower limit and upper limit, respectively, of this molar fraction.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **PAW ANDERSEN.** Algorithm for méthane number dermination for natural gasses. *Project Report,* 03 Juin 1999, vol. 719, 45

- **RACHFORD JR, H. H. ; RICE, J. D.** Procedure for use of electronic digital computers in calculating flash vaporisation hydrocarbon equilibrium. *Journal of Petroleum Technology,* 1952, vol. 4 (10), 19-3